# EUROPEAN PATENT SPECIFICATION

(11) **EP 1 978 935 B1**
(45) Date of publication and mention of the grant of the patent: **07.01.2015**
(21) Application number: 07702932.0
(22) Date of filing: 22.01.2007
(51) Int. Cl.: A61K 9/16, A61K 9/50, A61K 31/137

(54) **COATED FORMULATIONS**
BESCHICHTETE FORMULIERUNGEN
FORMULES ENROBÉES

(30) Priority: 23.01.2006 EP 06001314
(43) Date of publication of application: 15.10.2008
(73) Proprietor: LEK Pharmaceuticals d.d., 1526 Ljubljana (SI)
(72) Inventor: LEGEN, Igor, 1290 Grosuplje (SI); KUHAR, Polonca, 1000 Ljubljana (SI)
(74) Representative: Prüfer & Partner GbR European Patent Attorneys
(86) International application number: PCT/EP2007/000515
(87) International publication number: WO 2007/082770

(56) References cited:
- WO-A-03/053428
- WO-A-2004/105735
- WO-A-2005/079748
- WO-A-2005/097064
- WO-A-2007/011131

## Description

### FIELD OF THE INVENTION

The present invention relates to the field of pharmaceutics, and specifically to pharmaceutical formulation comprising a coating exhibiting a pH-dependent dissolution, characterized by the rapid dissolution at acidic conditions (which means at pH < 5.5, preferably < 5, more preferably at pH in stomach) and very slow dissolution at higher (meaning more alkaline than 5,5, preferably 6) pH values.

### BACKGROUND OF THE INVENTION

Many active pharmaceutical ingredients (drugs thereon) exhibit pH-dependent dissolution and/or pH-dependent stability. For these drugs and formulations thereof the change of the gastric pH can significantly affect the release of the drug from the formulation, which results in the altered pharmacokinetics and consequently altered pharmacodynamics of the drug. In the case of the increased plasma concentration of the drug the patient may experience enhanced side effects, while decreased plasma concentrations could result in the lack of the efficacy of the drug.

One of the most frequent change of the gastric pH is the elevation of the gastric pH caused by antacids. Antacids, including specifically group consisting of: magnesium carbonate, calcium carbonate, sodium bicarbonate, magnesium hydroxide, magnesium oxide, aluminum carbonate, aluminum hydroxide, aluminum phosphate, magnesium trisilicate, megaldrate, almagate, dihydroxyaluminum aminoacetate, and dihydroxyaluminum sodium carbonate (or mixtures thereof) work by neutralizing the excess of the stomach acid. For example, it was demonstrated that orally taken almagate (magnesium carbonate-aluminium hydroxide) increased gastric pH from about 1.2 to about 6.0 in about 5.5 minutes (Aliment. Pharmacol. Ther., 20, 683-688, 2004). Antacids are widely used for the relief of the gastro-oesopahageal reflux symptoms which show a high prevalence in general population (30-60%), antacids are also used for the treatment of duodenal ulcers, gastric ulcers, stress gastritis, bile acid mediated diarrhea, biliary reflux, constipation (Drugs, 57, 855-870, 1999) thus one can expect frequent concomitant use of antacids with other medications, including drugs and pharmaceutical formulations with pH-dependent dissolution and or pH-dependent stability.

A condition that is very frequently connected to the concomitant use of antacids with other medications is for example stress. It is noted that stress contributes to the etiology of between 30 % and 65 % of peptic ulcer cases (Psychosom. Med., 62, 176-185, 2000), and antacids are commonly used for the treatment of upper gastrointestinal ulcers (Drugs, 57, 855-870, 1999). It is also noted that stress is related to several diseases, including urinary incontinence (Int. J. Gynaecol. Obstet., 82, 327-338, 2003), heart and coronary diseases (Curr. Opin. Cardio.I, 19, 494-499, 2004), hypertension (Metabolism, 51, 40-45, 2002), Alzheimer's disease, Parkinson's disease, cancer, chronic renal disease, chronic locomotor system diseases, chronic intestinal diseases, chronic liver disease, gallbladder disease, polycystic ovary syndrome, prostatic hyperplasia, type 2 diabetes, infections (Clin. Nutr, 23, 1256-1266, 2004).

Drugs with the decreased plasma concentrations defined by the decreased peak plasma concentration (C_{MAX}) and/or area under the curve (AUC) due to increase of the gastric pH and consequently impaired *in vivo* dissolution are for example poorly-water soluble weakly basic drugs. Specifically: antifungal agent ketoconazole, anti-emetic agent cinnarizine, antibacterial agents enoxacin and cefpodoxime proxetil, antianxiety agent diazepam. Additionally, calcium salts and zinc salts also exhibit impaired *in vivo* dissolution due to elevated gastric pH. On the other hand a drug with the increased plasma concentrations defined by the increased C_{MAX} due to the concomitant use of antacids and consequently the increased *in vivo* dissolution of the drug is for example an urinary antispasmodic agent tolterodine in its tartrate salt form, thereon term tolterodin will comprise tolterodin or any salt or metabolite thereof, preferably tolterodine tartrate.

Decreased or increased as used above relates to values compared to the ones obtained by administering the drugs to patients having normal stomach and gastrointestinal pH.

Elevated gastric pH may also afford the enhanced absorption of the acid-labile drugs, which may be preferably: penicillins, erythromycin, used as antiinfectives or digoxin used to treat congestive heart failure or proton pump inhibitors such as omeprazole, lansoprazole, pantoprazole, enantiomers and salts thereof.

Elevated gastric pH might also change the pharmacokinetic properties of the coated formulations (i.e. enteric coated ketoprofen tablets), because the pH in the stomach increase as a result of the concomitant use of the antacids to the values that are characteristic for the small intestine, thus the release of the drug may begin already in the stomach, resulting in shorter T_{MAX} (time to reach maximum concentration) and increased C_{MAX}.

The high probability of impaired gastric pH associated with diseases and conditions selected form the above-mentioned groups, particularly urinary diseases, thus requires that drugs used for the manufacturing of the medicament for treatment of above diseases and/or conditions are formulated into a pharmaceutical composition exhibiting predictable pH dependant dissolution characteristics; especially if they are to be used concomitantly.

### DISCLOSURE OF THE INVENTION

The subject-matter of the invention is summarized as:
A coated pharmaceutical composition comprising an active pharmaceutical ingredient, wherein the active pharmaceutical ingredient is tolterodine or a salt thereof, wherein the coating of said composition comprises a polymer or copolymer of acrylate and/or methacrylate with ammonium group, and/or chitosan or chitosan derivatives, characterized by the rapid dissolution at acidic conditions (pH < 5.5) and slow dissolution at higher pH values.

The pharmaceutical composition as above wherein said active pharmaceutical ingredient has increased, plasma concentrations if administered concomitantly with an antacid.

A pharmaceutical composition comprising tolterodin tartrate, said composition having a coating as defined above with the pH-dependent dissolution, characterized by the rapid dissolution at pH < 5.5 and slow dissolution at higher pH values.

The pharmaceutical composition as above wherein the coating comprises a polymer or copolymer of dimethylaminoethyl methacrylate.

The pharmaceutical composition as above wherein the rapid dissolution at pH < 5.5 is defined by that at pH 2.0 the coating is completely dissolved in less than 30 minutes if subjected to the dissolution test in apparatus 2 in accordance with USP 28.

The pharmaceutical composition as above wherein slow dissolution at pH values above 5.5 is defined by that at pH 6.8 the coating is substantially not dissolved after 180 minutes if subjected to the dissolution test in apparatus 2 in accordance with USP 28.

The pharmaceutical composition as above characterized in that that less than 20% by weight of active pharmaceutical ingredient is released at pH = 6,8 within 3 h if subjected to the dissolution test in apparatus 2 in accordance with USP 28.

The pharmaceutical composition as above characterized in that that in the solution or the suspension less than 20% by weight of the amount of polymer used in coating is detected after 3 h, when subjecting the composition to dissolution test in apparatus 2 in accordance with USP 28.

The pharmaceutical composition as above, wherein the amount of polymer is detected by HPLC in solution or dispersion after subjecting the composition to dissolution test in apparatus 2 in accordance with USP 28.

The pharmaceutical composition as above, characterized in that it consists of cores coated with a first coating wherein cores comprise the above-mentioned active pharmaceutical ingredient, and a second separating coating and a third coating comprising copolymer of acrylate and methacrylate with ammonium group and the pH-dependent dissolution as defined above.

The pharmaceutical composition as above wherein the second coating comprises hydroxypropyl cellulose.

The pharmaceutical composition as above where the cores are pellets.

The pharmaceutical composition as above where the cores are inert cores coated with film comprising active pharmaceutical ingredient.

A pharmaceutical composition, characterized in that it consists of pellets which comprise tolterodine tartrate which are coated by one or optionally more coatings and at least one of said coatings comprises a polymer or copolymer of acrylate and/or methacrylate with quarternary ammonium group, the coating having the pH-dependent dissolution as defined above.

The pharmaceutical composition as above wherein said a polymer or copolymer is Eudragit E PO or Eudragit E 100.

A pharmaceutical composition, characterized in that it consists of pellet cores comprising tolterodine tartrate coated with a first coating, a second coating and a third coating comprising Eudragit E PO, sodium lauryl sulfate, stearic acid, and magnesium stearate with the pH-dependent dissolution as defined above.

The pharmaceutical composition as above characterized in that a second coating comprises ethylcellulose, and hydroxypropyl cellulose, while first coating may be enteric coating.

Use of the coating, the coating being comprised as defined above, characterized by the pH-dependent dissolution which is rapid at acidic conditions (pH < 5.5) and very slow at higher pH values for neutralizing the effect of the elevated pH on the release and/or stability of a drug and/or drug formulations that exhibit pH-dependent dissolution and/or stability, caused by the concomitant use of the antacids, wherein the drug is tolterodine or a salt thereof.

Use of coating as above in manufacturing a medicament comprising the aforementioned drug and exhibiting increased Cmax if administered concomitantly with an antacids.

Use of coating as above in manufacturing a medicament as defined above for treating urinary or gastrointestinal disease or hypertension.

Use of coating as above in manufacturing a medicament comprising tolterodine tartrate.

Use of a copolymer of acrylate and methacrylate with quarternary ammonium group and the pH-dependent dissolution as defined above for coating a pharmaceutical composition comprising tolterodin.

A process for manufacturing a pharmaceutical composition characterized in that it consists of following steps:
a) preparing cores comprising an active pharmaceutical ingredient (wherein said active pharmaceutical ingredient may be in or on cores), wherein the active pharmaceutical ingredient is tolterodine or a salt thereof;
b) applying a coating to said cores, the coating being comprised as defined above, characterized in that the coating layer provides for the rapid dissolution at pH < 5.5 and slow dissolution at higher pH values.

A process as above, wherein active pharmaceutical ingredient is tolterodin or tolterodine tartrate and the coating applied in step b) comprises aminoylkyl methacrylate copolymers and/or chitosan or chitosan derivatives and is characterized by the rapid dissolution at acidic conditions (pH < 5.5) and slow dissolution at higher pH values.

A process for manufacturing a pharmaceutical composition characterized in that it consists of following steps:
a) preparing cores comprising an active pharmaceutical ingredient (wherein said active pharmaceutical ingredient may be in or on cores), wherein the active pharmaceutical ingredient is tolterodine or a salt thereof; the cores may be prepared by mixing active pharmaceutical ingredient with inactive ingredients and granulating or extrusion or spheronization or may be prepared by applying a film containing active pharmaceutical ingredient into inert cores;
b) applying an enteric coating or release controlling agent to said cores;
c) applying second separating coating to said first coated cores, giving second cores;
d) applying third coating to said second cores, the coating comprising a polymer or copolymer of acrylate and/or methacrylate with ammonium group, and/or chitosan or chitosan derivatives, characterized in that third coating layer provides for the rapid dissolution at pH < 5.5 and slow dissolution at higher pH values.

A process in as above where the coating in step d) comprises a polymer selected from aminoalkyl methacrylate copolymers.

A process in as above wherein the aminoalkyl methacrylate copolymer is a copolymer of acrylate and methacrylate with quarternary ammonium group.

A process in as above where the coating in step c) comprises hydroxypropyl cellulose.

A process in as above, wherein the active pharmaceutical ingredient is tolterodine tartrate.

A composition as above for use in the treatment of a patient who is being concomitantly treated with antacid.

Use of coating comprising a polymer or copolymer of acrylate and/or methacrylate with ammonium group, and/or chitosan or chitosan derivatives, with the pH-dependent dissolution, characterized by the rapid dissolution at acidic conditions (pH < 5.5) and very slow dissolution at higher pH values for manufacturing a medicament, wherein the medicament comprises tolterodin or salt thereof.

Use as above for manufacturing a medicament as mentioned above for treating an urinary difficulty.

Use as above where urinary difficulty is frequent urination and/and inability to control urination

A composition as described or exemplified above for use in treating urinary diseases.

A composition as above, wherein the urinary disease is a urinary difficulty.

A composition as above, wherein the urinary difficulty is frequent urination and/and inability to control urination

A composition as described or exemplified above for use in a treatment by administering an urinary antispasmodic medicament, wherein the medicament comprises tolterodin or salt thereof, wherein the administration is preformed concomitantly with administration of an antacid.

The present invention is in a broad sense a pharmaceutical composition will comprise tolterodin or salt thereof as an active pharmaceutical ingredient, and a coating comprising a polymer or copolymer of acrylate and/or methacrylate with ammonium group, and/or chitosan or chitosan derivatives, characterized by the rapid dissolution at acidic conditions (pH < 5.5) and slow dissolution at higher pH values. Said active pharmaceutical ingredient may have increased plasma concentrations if administered concomitantly with an antacid.

In a specific aspect the invention is a pharmaceutical composition comprise tolterodin or salt thereof (in a specific embodiment comprising tolterodin tartrate), said composition having a coating providing for the pH-dependent dissolution, characterized by the rapid dissolution at pH < 5.5 and slow dissolution at higher pH values, in particular the formulations and coating thereof which comprises a polymer or copolymer of acrylate and/or methacrylate with quarternary ammonium group, such as dimethylaminoethyl methacrylate (Eudragit E PO).

In one embodiment of the specified pharmaceutical composition of the invention, the rapid dissolution at pH < 5.5 is defined by that at pH 2.0 the coating is completely dissolved in less than 30 minutes if subjected to the dissolution test in apparatus 2 in accordance with USP 28 and/or the slow dissolution at pH values above 5.5 is defined by that at pH 6.8 the coating is substantially not dissolved after 180 minutes if subjected to the dissolution test in apparatus 2 in accordance with USP 28. Specifically this can be detected by fact that that in the solution or the suspension less than 20% by weight of the amount of polymer used in coating is detected, (which can be performed by HPLC analysis) after 3 h from the start of dissolution test, when subjecting the composition to dissolution test in apparatus 2 in accordance with USP 28.

In an alternative embodiment the dissolution characteristics of said composition are characterized in that that less than 20% by weight of the afore-mentioned active pharmaceutical ingredient is released into solution at pH = 6,8 within 3 h (from the start of dissolution test) if subjected to the dissolution test in apparatus 2 in accordance with USP 28.

The pharmaceutical composition of the present invention in one structural aspect and preferred embodiment consists of cores coated with a first coating wherein cores comprise the specified active pharmaceutical ingredient, and a second separating coating and a third coating comprising copolymer of acrylate and methacrylate with quarternary ammonium group, preferably wherein the second coating comprises hydroxypropyl cellulose, where as specified above the active pharmaceutical ingredient is tolterodin, or salt thereof, more preferably where the coated cores coated are pellets, which are filled into capsule or compressed into tablets.

In a specific aspect the invention is a pharmaceutical composition, characterized in that it consists of pellet which comprise tolterodine tartrate which are coated by one or optionally more coatings and at least one of said coatings comprises a polymer or copolymer of acrylate and/or methacrylate with quarternary ammonium group, preferably Eudragit E PO or Eudragit E 100.

In yet more specific aspect the invention is a pharmaceutical composition, characterized in that it consists of pellet cores comprising the specified active pharmaceutical ingredient for treating a stress related disease, which may be urinarly disease, gastrointestinal disease, hypertension, (in particular tolterodine tartrate for treating urinary disease), coated with a first coating, a second coating and a third coating comprising Eudragit E PO, sodium lauryl sulfate, stearic acid, and magnesium stearate, preferably where second coating comprises ethylcellulose, and/or hydroxypropyl cellulose, while first coating (which may be in certain embodiments optional) may comprise an anionic polymer containing alkaline functional groups.

In another specific structural embodiment the cores may be inert cores such as sugar spheres onto which active pharmaceutical ingredient is applied as film.

In the particular embodiment the present invention is a finished dosage form (that is a pharmaceutical composition in a form suitable for the administration to patients) comprising the pharmaceutical composition as specified above and according to any of the attached claims filled into capsules, where in case gelatine or HPMC capsules are used it has been found those have no effect on pH dependant release

Skilled person will appreciate different structural approaches in order to achieve desired pH dependant dissolution. Such structures may be for example pellets providing for controlled release which are incorporated into multiple units tablet coated with a coating which provides the effect in accordance with the invention or such pellets or a structurally different reservoir devices incorporated into a releasing device which releases said reservoir device or active ingredient contained therein in controlled pH dependant matter.

Use of the coating characterized by the pH-dependent dissolution which is rapid at acidic conditions (pH < 5.5) and very slow at higher pH values for neutralizing the effect of the elevated pH on the release and/or stability of the drug and/or drug formulations (wherein the drug is as specified above) that exhibit pH-dependent dissolution and/or stability, caused by the concomitant use of the antacids is contemplated within the scope of the invention, in particular the use of a copolymer of acrylate and methacrylate with quarternary ammonium group for coating a pharmaceutical composition comprising tolterodin.

The invention is for example embodied in a process for manufacturing a pharmaceutical composition characterized in that it consists of following steps:
a) preparing cores comprising the specified active pharmaceutical ingredient;
b) applying a coating to said cores, characterized in that the coating layer comprises aminoylkyl methacrylate copolymers and/or chitosan or chitosan derivatives and provides for the rapid dissolution at pH < 5.5 and slow dissolution at higher pH values..

More specifically the invention is embodied in a process for manufacturing a pharmaceutical composition characterized in that it consists of following steps:
a) Preparing cores comprising the specified active pharmaceutical ingredient;
b) applying an enteric coating or release controlling agent to said cores;
c) applying second separating coating to said first coated cores, giving second cores;
d) applying third coating to said second cores, characterized in that third coating layer provides for the rapid dissolution at pH < 5.5 and slow dissolution at higher pH values and comprises a polymer selected from aminoalkyl methacrylate copolymers, (preferably a copolymer of acrylate and methacrylate with quarternary ammonium group), chitosan or chitosan derivatives, in particular also the coating in step c) comprises a cellulose derivative such as and preferably hydroxypropyl cellulose.

The aspect of the invention are also the use of the above described composition for treatment of patient who is being concomitantly treated with antacid.

In summary the general aspect of the invention is thus the use of coating with the pH-dependent dissolution comprising a polymer or copolymer of acrylate and/or methacrylate with ammonium group, and/or chitosan or chitosan derivatives, characterized by the rapid dissolution at acidic conditions (pH < 5.5) and very slow dissolution at higher pH values for manufacturing a medicament comprising tolterodine or a salt thereof.

According to the present invention the specified drugs are exemplified to be used for stress related diseases, in particular urinary disease. The medicament comprises tolterodin or a salt thereof and the use of so coated compositions for manufacturing a medicament for treating an urinary difficulty, which is preferably a frequent urination and/and inability to control urination.

Treating urinary diseases is by administering above described compositions, in particular treating urinary difficulty.

Yet another aspect of the invention is a use in a treatment of administering urinary antispasmodic medicament, by using a described composition wherein the administration is preformed concomitantly with administration of an antacid.

### DETAILED DESCRIPTION OF THE INVENTION

The present invention describes the use of the above-specified coating in the manufacturing of the specified pharmaceutical composition with the pH-dependent dissolution, characterized by the rapid dissolution thereof at acidic conditions, that is at pH < 5.5; which means that at pH 2.0 the coating comprising a coating agent is completely dissolved in less than 30 minutes, preferably in less than 20 minutes, more preferably in less than 15 minutes (if coating dissolution is measured under sink conditions in an USP 28 dissolution apparatus 2) and slow dissolution at pH values higher than 5.5 that is at pH 6.8 the coating is substantially not dissolved even after 180 minutes (if coating dissolution is measured under sink conditions in an USP 28 dissolution apparatus 2). The fact that coating is substantially not dissolved may be characterized in one embodiment by measuring the release active pharmaceutical ingredient (API) wherein the release of the above-mentioned API is not a limited or modified factor, in that at pH = 6,8 (0.1 M phosphate buffer, USP 28 apparatus 2) less than 30% of API is released within 3 h, preferably less than 20% after 1 h; more preferably less than 10% after 2 h. In the other embodiment it may be characterized by the fact that in the solution or the suspension (when subjecting the coated composition to dissolution test in apparatus 2 in accordance with USP 28) less than 20% by weight of the amount of polymer (coating agent) used in coating is detected after 3 h, preferably less than 10% after 1 h, more preferably less than 10% after 2 h, still more preferably less than 10 after 3 h; which can be conveniently measured spectrofotometrically or alternatively by HPLC or MS or other analytical technique.

It is assumed that the dissolution of the specified API is not a limiting factor. The invention is thus particularly suitable for the form of salts.

This afore-mentioned coating is intended to neutralize the effect of the elevated pH on the release/stability of the specified drug and drug formulations that exhibit pH-dependent dissolution and/or stability, in particular caused by the concomitant use of the antacids and is applied in the pharmaceutical composition in a manner that it comes in the contact with the gastric content sooner then the drug. It is contemplated that the coating primarily influences the pH dependence of the dissolution and/or stability characteristics of the drugs and/or pharmaceutical compositions, however the cores being coated may be also formulated (manufactured) in a way to provide modified release after the coating has dissolved or disintegrated.

Coating contains a polymer or combination of polymers which contain alkaline functional groups as defined above. Polymers are selected from the purely synthetic materials such as aminoalkyl methacrylate copolymers or from the natural polymers such as chitosan and chitosan derivatives. Under the normal acidic conditions in the stomach this coating is rapidly dissolved without affecting the release of the specified drug from the pharmaceutical formulation. If antacids are concomitantly used with the specified drug and pharmaceutical formulation that exhibit pH-dependent dissolution and/or stability the above mentioned coating prevents the release of the drug from the pharmaceutical formulation and thus neutralize the effect of elevated pH on the drug release/stability. When pH in the stomach falls under the pH at which the above mentioned coating is dissolved (i.e. in the case of orally taken almagate the gastric pH, after being increased to about 6.0 in about 5.5 minutes, decreased under 5.5 in about 10 minutes (Aliment. Pharmacol. Ther., 20, 683-688, 2004)) as a result of the ceasing of the antacid activity, the coating is dissolved and the drug or pharmaceutical formulation is exposed to more normal acidic conditions in the stomach, enabling the more predictive pharmacokinetic and consequently more predictive efficacy of the specified drug.

### Formulation to avoid enhanced absorption of the specified drug

In the case of the specified acid-labile drug, elevated gastric pH may result in enhanced absorption and subsequently enhanced adverse effects. This undesired effect can be circumvented by coating the cores, with a coating layer of a coating agent, preferably an anionic polymer containing alkaline functional groups, more preferably an acrylate polymer, most preferably copolymer of acrylate and methacrylates with quarternary ammonium group, such as Eudragit E PO.

The cores may be either crystals, granules, pellets, tablets or capsules comprising above mentioned drug and suitable pharmaceutical excipients. Specifically the cores may be pellets or granules comprising the specified API therein or alternatively inert cores, such as sugar spheres onto which the specified API is applied as a film.

The following table shows a typical weight (relative to finished composition) composition of an above-mentioned coating to be applied to the cores of the pharmaceutical composition mentioned above, whereas the coating amounts to 10-20 % by weight to the finished composition.

| | | |
|---|---|---|
| coating agent | specified polymer (preferably: Eudragit E PO) | 6,0-12,0 |
| surfactant | sodium lauryl sulfate, polysorbate 80 | 0,5-1,5 |
| plasticizer | stearic acid, dibutyl sebacate, acetyltributyl citrate | 0,5-1,5 |
| glidant | magnesium stearate, talc | 2,0-5,0 |

Cores, preferably pellets are prepared, preferably by extrusion and spheronisation of wet mass consisting of the specified API, sufficient amount of spheronising agent (i. e. microcrystalline cellulose), optionally release controlling agent and granulation fluid (i.e. water, ethanol). Dry cores are coated with the coating dispersion prepared in following steps. First, surfactant, preferably a sodium lauryl sulfate, is dissolved in purified water and plasticizer. preferably containing stearic acid and/or dibutyl sebacate and/or acetyltributyl citrate or similar excipient, preferably stearic acid, added while stirring (e.g. by propeller mixer). Then, coating agent is dispersed into prepared liquid and mixed (e.g. with high shear mixer) until clear, yellowish dispersion is formed. Separately, suspension of glidant in water is prepared, added to polymer dispersion and mixed. The resulting dispersion is used for coating of the cores in a fluid-bed device.

### Formulation to avoid premature release of coated composition

Due to elevated gastric fluid pH changes in the pharmacokinetic properties of coated, preferably enteric coated, formulations may occur and the release of the specified drug may begin already in the stomach. Additional coating layer comprising of polymer with cationic properties is able to prevent this undesired effect. Due to possible interactions between anionic polymer for first coating and cationic polymer for protective coating additional inert separating coating layer may be required. First coated material may be in the form of coated crystals, granules, pellets, tablets or capsules.

The following table shows a typical weight (relative to finished composition) composition of a separating (second) coating to be applied to the preferably enteric coated cores of the pharmaceutical composition, whereas the coating amounts to 3 -30 % by weight to the finished composition. Composition will depend on the first and third coating.

| | | |
|---|---|---|
| coating agent | hydroxypropyl cellulose | preferably 2-5 |
| plasticizer | polyethylene glycol | preferably 0,2-0,5 |
| glidant | talc | preferably 0,5-1,0 |

Bellow is a typical weight (relative to finished composition) composition of a (third) coating to be applied to the cores of the pharmaceutical composition, whereas the coating amounts to 10 - 20 % by weight to the finished composition.

| | | |
|---|---|---|
| coating agent | specified polymer (preferably: Eudragit E PO) | 6,0-12,0 |
| surfactant | sodium lauryl sulfate | 0,5-1,5 |
| plasticizer | stearic acid | 0,5-1,5 |
| glidant | magnesium stearate | 2,0-5,0 |

Cores, preferably pellets are prepared by extrusion and spheronisation of wet mass consisting of active substance, sufficient amount of spheronising agent (e.g. microcrystalline cellulose), optionally release controlling agent and granulation fluid (i.e. water, ethanol).

Dry cores are first coated with separating layer, prepared in following steps: (a) coating agent, preferably powdered hydroxypropyl cellulose, but also hydroxypropylmethylcellulose or methylcellulose is slowly added to well-agitated liquid, preferably water at room temperature and stirred until a gel-free solution is obtained, (b) plasticizer, preferably polyethylene glycol, but also glycerol, propylene glycol, triacetin is stirred into solution, (c) glidant, preferably talc suspension in suitable liquid, preferably water, is prepared separately (e.g. with high-shear mixing) and (d) combined with above solution. Cores are coated and preferably dried in a fluid bed device. Finally, the protective coating is applied on coated cores. The coating dispersion is prepared in following steps: (a) surfactant, preferably sodium lauryl sulphate but also a poloxamer, is dissolved (e.g. in purified water) and a plasticizer, preferably stearic acid, is added while stirring (e.g. with propeller mixer), (b) coating agent (a polymer as specified above) is dispersed into prepared liquid and mixed (e.g. with high shear mixer) until clear dispersion is formed Separately, suspension of glidant, preferably magnesium stearate or also talc or glycerol monostearat, (e.g. in water) is prepared (c), added to above dispersion and mixed. The resulting dispersion (d) is used for coating of the cores in a fluid-bed device.

### Working Example 1

| **Pellet cores (weight per capsule = 160 mg)** | |
|---|---|
| Tolterodine tartrate | 4.00 mg |
| Microcrystalline cellulose | 156.00 mg |
| Purified water | 180.00 mg |

| **Coating (15 % application, based on coated pellets weight)** | |
|---|---|
| Eudragit E PO | 15,00 mg |
| Sodium lauryl sulfate | 1,50 mg |
| Stearic acid | 2,00 mg |
| Magnesium stearate | 6,00 mg |
| Purified water | 140,0 mg |

Tolterodine tartrate (API) and microcrystalline cellulose are mixed and granulated with water. Granulate is coated by a dispersion prepared as follows: sodium lauryl sulphate is dissolved in purified water and stearic acid is added while stirring, thereto the above-mentioned polymer is dispersed and mixed dispersion is formed. Thereto separately prepared suspension of magnesium stearate in water is added to said polymer and the resulting dispersion is used for coating.

### Working Example 2

| **Pellet cores (weight per capsule = 160 mg)** | |
|---|---|
| Tolterodine tartrate | 4.000 mg |
| Microcrystalline cellulose | 156.000 mg |
| Purified water | 180.000 mg |

| **Separating coating (15 % application based on core weight)** | |
|---|---|
| Ethylcellulose | 26,253 mg |
| Hypromellose | 2,172 mg |
| Purified water | 110,769 mg |

| **Coating (10 % application, based on coated pellets weight)** | |
|---|---|
| Eudragit E PO | 13,07 mg |
| Sodium lauryl sulfate | 1,31 mg |
| Stearic acid | 1,96 mg |
| Magnesium stearate | 4,57 mg |
| Purified water | 118,52 mg |

Tolterodine tartrate and microcrystalline cellulose are mixed. Demineralised water is added and mixed. From the homogeneous blend, pellet cores are made using the method of extrusion and spheronization. Coating dispersion is prepared in following steps. First, ethylcellulose polymer dispersions with purified water is prepared. Separately, hypromellose is dissolved in water, added to ethylcellulose dispersion and mixed. The resulting dispersion is used for coating of the pellet cores in a fluid-bed device.

Subsequnetly the protective coating is applied on dry coated pellets. The coating dispersion is prepared in following steps: (a) sodium lauryl sulphate is dissolved in purified water and stearic acid is added while stirring with propeller mixer, (b) specified polymer is dispersed into prepared liquid and mixed with high shear mixer until clear, yellowish dispersion is formed. Separately, suspension of magnesium stearate in water is prepared (c), added to said polymer dispersion and mixed. The resulting dispersion (d) is used for coating of the pellet cores in a fluid-bed device.

### Working Example 3

| | |
|---|---|
| **Pellet cores (weight per capsule = 160 mg)** | |
| Tolterodine tartrate | 4.00 mg |
| Microcrystalline cellulose | 156.00 mg |
| Purified water | 180.00 mg |
| **Coating (20 % application, based on coated pellets weight)** | |
| Dimethylaminoethyl methacrylate (Eudragit E 100) | 32,40 mg |
| Polyethylene glycol 6000 | 2,00 mg |
| Talc | 7,60 mg |
| Isopropyl alcohol | 212,40 mg |
| Acetone | 141,60 mg |
| Purified water | 4,00 mg |

Tolterodine tartrate and microcrystalline cellulose are mixed. Demineralised water is added and mixed. From the homogeneous blend, pellet cores are made using the method of extrusion and spheronization. Coating dispersion is prepared in following steps. One part (2/3) of Isopropyl alcohol and acetone are introduced first and then Eudragit E 100 is added while stirring with a propeller stirrer for aprox. 30-60 minutes until a clear solution is formed. Separately, polyethylene glycol 6000 is dissolved in water and slowly added to remaining isopropyl alcohol. Then, talc is added and suspension is homogenized for 20 minutes. Finally, the talc suspension is added to the polymer solution with gentle stirring.

The coating suspension is applied to the pellet cores in fluid bed device.

### Working Example 4

| **Pellet cores (weight per capsule = 160 mg)** | |
|---|---|
| Tolterodine tartrate | 4.000 mg |
| Microcrystalline cellulose | 156.000 mg |
| Purified water | 180.000 mg |

| **Separating coating (15 % application based on core weight)** | |
|---|---|
| Ethylcellulose | 26,253 mg |
| Hypromellose | 2,172 mg |
| Purified water | 110,769 mg |

| **Coating (20 % application, based on coated pellets weight)** | |
|---|---|
| Eudragit E PO | 29,41 mg |
| Sodium lauryl sulfate | 2,94 mg |
| Stearic acid | 4,42 mg |
| Magnesium stearate | 10,33 mg |
| Purified water | 247,01 mg |

Tolterodine tartrate and microcrystalline cellulose are mixed. Demineralised water is added and mixed. From the homogeneous blend, pellet cores are made using the method of extrusion and spheronization. Coating dispersion is prepared in following steps. First, ethylcellulose polymer dispersions with purified water is prepared. Separately, hypromellose is dissolved in water, added to ethylcellulose dispersion and mixed. The resulting dispersion is used for coating of the pellet cores in a fluid-bed device.

Subsequnetly the protective coating is applied on dry coated pellets. The coating dispersion is prepared in following steps: (a) sodium lauryl sulphate is dissolved in purified water and stearic acid is added while stirring with propeller mixer, (b) specified polymer is dispersed into prepared liquid and mixed with high shear mixer until clear, yellowish dispersion is formed. Separately, suspension of magnesium stearate in water is prepared (c), added to polymer dispersion and mixed. The resulting dispersion (d) is used for coating of the pellet cores in a fluid-bed device.

### Working Example 5

Pellet cores of Example 4 are replaced by sugar spheres onto which a film containing active pharmaceutical ingredient as disclosed below is applied in fluid bed device. The resulting cores are after drying to form and cure film coated with separating and final coating as in Example 4.

| **Film applied to sugar spheres (weight per capsule = 160 mg)** | |
|---|---|
| Tolterodine tartrate | 4.000 mg |
| Polyvinylpyrrolidone | 20.000 mg |
| Dibutylsebacate | 2.000 mg |
| Polysorbate 80 | 0.100 mg |
| Water | |

## Claims

1. A pharmaceutical composition comprising an active pharmaceutical ingredient, wherein the active pharmaceutical ingredient is tolterodine or a salt thereof, said composition having a coating comprising a polymer or copolymer of acrylate and/or methacrylate with ammonium group, and/or chitosan or chitosan derivatives, **characterized by** the rapid dissolution at acidic conditions (pH < 5.5) and slow dissolution at higher pH values.

2. A pharmaceutical composition in accordance with claim 1 comprising tolterodine tartrate, said composition having a coating with the pH-dependent dissolution, **characterized by** the rapid dissolution at pH < 5.5 and slow dissolution at higher pH values.

3. The pharmaceutical composition in accordance with claim 2, wherein the coating comprises a polymer or copolymer of acrylate and/or methacrylate with ammonium group.

4. The pharmaceutical composition in accordance with claim 3, wherein the coating comprises a polymer or copolymer of dimethylaminoethyl methacrylate.

5. The pharmaceutical composition in accordance with any of the claims 1 to 4, wherein the rapid dissolution at pH < 5.5 is defined by that at pH 2.0 the coating is completely dissolved in less than 30 minutes if subjected to the dissolution test in apparatus 2 in accordance with USP 28.

6. The pharmaceutical composition in accordance with any of the claims 1 to 4, wherein slow dissolution at pH values above 5.5 is defined by that at pH 6.8 the coating is substantially not dissolved after 180 minutes if subjected to the dissolution test in apparatus 2 in accordance with USP 28.

7. The pharmaceutical composition according to any of the claims 1 to 4, **characterized in that** that less than 20% by weight of active pharmaceutical ingredient is released at pH = 6,8 within 3 h if subjected to the dissolution test in apparatus 2 in accordance with USP 28.

8. The pharmaceutical composition according to any of the claims 1 to 4, **characterized in that** that in the solution or the suspension less than 20% by weight of the amount of polymer used in coating is detected after 3 h, when subjecting the composition to dissolution test in apparatus 2 in accordance with USP 28.

9. The pharmaceutical composition according to claim 8, wherein the amount of polymer is detected by HPLC in solution or dispersion after subjecting the composition to dissolution test in apparatus 2 in accordance with USP 28.

10. The pharmaceutical composition according to any of the previous claims, **characterized in that** it consists of cores coated with a first coating wherein cores comprise said active pharmaceutical ingredient, and a second separating coating and a third coating comprising copolymer of acrylate and methacrylate with ammonium group.

11. The pharmaceutical composition according to previous claim wherein the second coating comprises hydroxypropyl cellulose.

12. The pharmaceutical composition according to claims 10 or 11 where the cores are pellets.

13. The pharmaceutical composition according to claim 10 or 11 where the cores are inert cores coated with film comprising active pharmaceutical ingredient.

14. A pharmaceutical composition, **characterized in that** it consists of pellet which comprise tolterodine tartrate which are coated by one or optionally more coatings and at least one of said coatings comprises a polymer or copolymer of acrylate and/or methacrylate with ammonium groups and/or chitosan or chitosan derivatives, the coating being **characterized by** a rapid dissolution at acidic conditions (pH < 5.5) and slow dissolution at higher pH values.

15. The pharmaceutical composition in accordance with claim 14, wherein said a polymer or copolymer is Eudragit E PO or Eudragit E 100.

16. A pharmaceutical composition, **characterized in that** it consists of pellet cores comprising tolterodine tartrate coated with a first coating, a second coating and a third coating comprising Eudragit E PO, sodium lauryl sulfate, stearic acid, and magnesium stearate, the coating being **characterized by** a rapid dissolution at acidic conditions (pH < 5.5) and slow dissolution at higher pH values.

17. The pharmaceutical composition according to claim 16, **characterized in that** a second coating comprises ethylcellulose, and/or hydroxypropyl cellulose.

18. A pharmaceutical composition comprising cores wherein cores comprise tolterodine tartrate,
and first coating which comprises
6-12% of coating agent;
0,5-1,5% of surfactant;
0,5-1,5 of plasticizer; and
2 - 5 % of glidant;
and separating coating which comprises
2-5% of coating agent;
0,2-0,5% of plasticizer; and
0,5-1% of glidant;
and third coating comprising
6-12% of coating agent, which is a polymer or copolymer of acrylate and/or methacrylate with ammonium groups;
0,5-1,5% of surfactant;
0,5-1,5% of plasticizer; and
2-5% of glidant;
whereby the amounts mean by weight to the finished composition, and wherein the third coating is **characterized by** a rapid dissolution at acidic conditions (pH < 5.5) and slow dissolution at higher pH values.

19. A pharmaceutical composition according to claim 18,
wherein in the first coating the coating agent is a polymer or copolymer of acrylate and/or methacrylate with ammonium groups; the surfactant is sodium lauryl sulfate or polysorbate 80; the plasticizer is stearic acid or dibutyl sebacate or acetyltributyl citrate; and the glidant is magnesium stearate or talc;
and wherein in the separating coating the coating agent is hydroxypropyl cellulose; the plasticizer is polyethylene glycol; and the glidant is talc;
and wherein in the third coating the surfactant is sodium lauryl sulfate; the plasticizer is stearic acid; and the glidant is magnesium stearate.

20. A pharmaceutical composition which is a finished dosage form comprising the pharmaceutical composition according to any of the previous claims filled into gelatine or HPMC capsules.

21. Use of a coating comprising a polymer or copolymer of acrylate and/or methacrylate with ammonium group, and/or chitosan or chitosan derivatives, **characterized by** a pH-dependent dissolution which is rapid at acidic conditions (pH < 5.5) and very slow at higher pH values for neutralizing the effect of the elevated pH on the release and/or stability of a drug and/or of drug formulations that exhibit pH-dependent dissolution and/or stability, caused by the concomitant use of the antacids, wherein the drug is tolterodine or a salt thereof.

22. Use of coating in accordance with claim 21 in manufacturing a medicament exhibiting increased Cmax if administered concomitantly with an antacids.

23. Use of coating in accordance with claim 22 in manufacturing a medicament for treating urinary or gastrointestinal disease or hypertension.

24. Use of coating in accordance with previous claim in manufacturing a medicament comprising tolterodine tartrate.

25. Use of a copolymer of acrylate and methacrylate with ammonium group for coating a pharmaceutical composition comprising tolterodine.

26. A process for manufacturing a pharmaceutical composition **characterized in that** it consists of following steps:
a) preparing cores comprising an active pharmaceutical ingredient, wherein the active pharmaceutical ingredient is tolterodine or a salt thereof;
b) applying a coating to said cores, wherein said coating comprises a polymer or copolymer of acrylate and/or methacrylate with ammonium group, and/or chitosan or chitosan derivatives, **characterized in that** the coating layer provides for the rapid dissolution at pH < 5.5 and slow dissolution at higher pH values.

27. A process according to previous claim, wherein active pharmaceutical ingredient is tolterodine or tolterodine tartrate and the coating applied in step b) comprises aminoylkyl methacrylate copolymers and/or chitosan or chitosan derivatives.

28. A process for manufacturing a pharmaceutical composition **characterized in that** it consists of following steps:
a) preparing cores comprising an active pharmaceutical ingredient, wherein the active pharmaceutical ingredient is tolterodine or a salt thereof,;
b) applying an enteric coating or release controlling agent to said cores;
c) applying second separating coating to said first coated cores, giving second cores;
d) applying third coating to said second cores, wherein said coating comprises a polymer or copolymer of acrylate and/or methacrylate with ammonium group, and/or chitosan or chitosan derivatives, **characterized in that** third coating layer provides for the rapid dissolution at pH < 5.5 and slow dissolution at higher pH values.

29. A process in accordance with claim 28 where the coating in step d) comprises a polymer, which is a aminoalkyl methacrylate copolymer.

30. A process in accordance with claim 29, wherein the aminoalkyl methacrylate copolymer is a copolymer of acrylate and methacrylate with ammonium group.

31. A process in accordance with any of the claims 28-30 where the coating in step c) comprises hydroxypropyl cellulose.

32. A process in accordance with any of the claims 28-31 wherein the active pharmaceutical ingredient is tolterodine tartrate.

33. A composition in accordance with any of claims 1 to 20 for use in the treatment of a patient who is being concomitantly treated with antacid.

34. The pharmaceutical composition in accordance with claim 1for use as a medicament in a treatment of patient who is being concomitantly treated with antacid.

35. The pharmaceutical composition for use in accordance with claim 34, wherein the medicament is for treating an urinary difficulty.

36. The pharmaceutical composition for use in accordance with claim 35, where urinary difficulty is frequent urination and/and inability to control urination.

37. The composition in accordance with any of claims 1 to 20 for use in treating urinary diseases.

38. The composition for use in accordance with claim 37, wherein the urinary disease is a urinary difficulty.

39. The composition for use in accordance with claim 38, wherein the urinary difficulty is frequent urination and/and inability to control urination.

40. The composition in accordance with any of claims 1 to 20 for use in administering urinary antispasmodic medicament, wherein the administration is preformed concomitantly with administration of an antacid.

## Patentansprüche

1. Pharmazeutische Zusammensetzung, welche einen aktiven pharmazeutischen Bestandteil umfasst, wobei der aktive pharmazeutische Bestandteil Tolterodin oder ein Salz davon ist, wobei die Zusammensetzung eine Beschichtung besitzt, welche ein Polymer oder ein Copolymer von Acrylat und/oder Methacrylat mit Ammoniumgruppe, und/oder Chitosan oder Chitosan-Derivate umfasst,
**gekennzeichnet durch** schnelle Auflösung bei sauren Bedingungen (pH < 5,5) und langsame Auflösung bei höheren pH-Werten.

2. Pharmazeutische Zusammensetzung gemäß Anspruch 1, umfassend Tolterodin-Tartrat, wobei die Zusammensetzung eine Beschichtung mit der pH-abhängigen Auflösung besitzt, **gekennzeichnet durch** die schnelle Auflösung bei einem pH < 5,5 und eine langsame Auflösung bei höheren pH-Werten.

3. Pharmazeutische Zusammensetzung gemäß Anspruch 2, wobei die Beschichtung ein Polymer oder Copolymer von Acrylat und/oder Methacrylat mit Ammoniumgruppe umfasst.

4. Pharmazeutische Zusammensetzung gemäß Anspruch 3, wobei die Beschichtung ein Polymer oder Copolymer von Dimethylaminoethylmethacrylat umfasst.

5. Pharmazeutische Zusammensetzung gemäß einem der Ansprüche 1 bis 4, wobei die schnelle Auflösung bei einem pH < 5,5 dadurch definiert ist, dass bei einem pH von 2,0 die Beschichtung in weniger als 30 Minuten komplett aufgelöst ist, wenn sie einem Auflösungstest in Apparat 2 nach USP 28 unterzogen wird.

6. Pharmazeutische Zusammensetzung gemäß einem der Ansprüche 1 bis 4, wobei langsame Auflösung bei pH-Werten oberhalb 5,5 dadurch definiert ist, dass bei einem pH von 6,8 die Beschichtung nach 180 Minuten im Wesentlichen nicht aufgelöst ist, wenn die Beschichtung einem Auflösungstest in Apparat 2 gemäß USP 28 unterzogen wird.

7. Pharmazeutische Zusammensetzung gemäß einem der Ansprüche 1 bis 4, **dadurch gekennzeichnet, dass** weniger als 20 Gew.-% von aktivem pharmazeutischem Bestandteil bei einem pH von 6,8 innerhalb von 3 Stunden freigesetzt wird, wenn sie dem Löslichkeitstest in Apparat 2 gemäß USP 28 unterzogen wird.

8. Pharmazeutische Zusammensetzung gemäß einem der Ansprüche 1 bis 4, **dadurch gekennzeichnet, dass** in der Lösung oder der Suspension weniger als 20 Gew.-% der Menge des beim Beschichten benutzten Polymers innerhalb von 3 Stunden detektiert wird, wenn die Zusammensetzung einem Auflösungstest in Apparat 2 gemäß USP 28 unterzogen wird.

9. Pharmazeutische Zusammensetzung gemäß Anspruch 8, wobei die Menge von Polymer durch HPLC in Lösung oder Dispersion detektiert wird, nachdem die Zusammensetzung einem Auflösungstest in Apparat 2 gemäß USP 28 unterzogen wurde.

10. Pharmazeutische Zusammensetzung gemäß einem der vorherigen Ansprüche, **dadurch gekennzeichnet, dass** sie aus Kernen, welche mit einer ersten Beschichtung beschichtet sind, wobei Kerne den aktiven pharmazeutischen Bestandteil umfassen, und einer zweiten trennenden Beschichtung und einer dritten Beschichtung, welche Copolymer von Acrylat und Methacrylat mit Ammoniumgruppe umfasst, besteht.

11. Pharmazeutische Zusammensetzung gemäß dem vorherigen Anspruch, wobei die zweite Beschichtung Hydroxypropylcellulose umfasst.

12. Pharmazeutische Zusammensetzung gemäß Anspruch 10 oder 11, wobei die Kerne Pellets sind.

13. Pharmazeutische Zusammensetzung gemäß Anspruch 10 oder 11, wobei die Kerne inerte Kerne sind, welche mit Film, welcher den aktiven pharmazeutischen Bestandteil umfasst, beschichtet sind.

14. Pharmazeutische Zusammensetzung, **dadurch gekennzeichnet, dass** sie aus Tolterodin-Tartrat umfassendem Pellet besteht, welche(s) mit einem oder wahlweise mehreren Beschichtungen beschichtet ist/ sind und mindestens eine der Beschichtungen ein Polymer oder Copolymer von Acrylat und/oder Methacrylat mit Ammoniumgruppen, und/oder Chitosan oder Chitosanderivate umfasst, wobei die Beschichtung durch eine schnelle Auflösung bei sauren Bedingungen (pH < 5,5) und eine langsame Auflösung bei höheren pH-Werten gekennzeichnet ist.

15. Pharmazeutische Zusammensetzung gemäß Anspruch 14, wobei das eine Polymer oder Copolymer Eudragit E PO oder
Eudragit E 100 ist.

16. Pharmazeutische Zusammensetzung, **dadurch gekennzeichnet, dass** sie aus Pelletkernen besteht, welche Tolterodin-Tartrat umfasst, welche mit einer ersten Beschichtung, einer zweiten Beschichtung und einer dritten Beschichtung, welche Eudragit E PO, Natriumlaurylsulfat, Stearinsäure und Magnesiumstearat umfasst, beschichtet sind, wobei die Beschichtung durch eine schnelle Auflösung bei sauren Bedingungen (pH < 5,5) und eine langsame Auflösung bei höheren pH-Werten gekennzeichnet ist.

17. Pharmazeutische Zusammensetzung gemäß Anspruch 16, **dadurch gekennzeichnet, dass** eine zweite Beschichtung Ethylcellulose und/oder Hydroxypropylcellulose umfasst.

18. Pharmazeutische Zusammensetzung, umfassend:
Kerne, wobei Kerne Tolterodin-Tartrat umfassen,
und erste Beschichtung, welche umfasst:
6-12% Beschichtungsmittel;
0,5-1,5% oberflächenaktiver Stoff;
0,5-1,5% Weichmacher; und
2-5% Gleitmittel;
und trennende Beschichtung, welche umfasst:
2-5% Beschichtungsmittel;
0,2-0,5% Weichmacher; und
0,5-1% Gleitmittel;
und dritte Beschichtung, welche umfasst:
6-12% Beschichtungsmittel, welches ein Polymer oder Copolymer von Acrylat und/oder Methacrylat mit Ammoniumgruppen ist;
0,5-1,5% oberflächenaktiver Stoff;
0,5-1,5% Weichmacher; und
2-5% von Gleitmittel;
wobei die Mengen an Gewicht auf die fertige Zusmmensetzung bezogen sind, und wobei die dritte Beschichtung durch eine schnelle Auflösung bei sauren Bedingungen (pH < 5,5) und eine langsame Auflösung bei höheren pH-Werten gekennzeichnet ist.

19. Pharmazeutische Zusammensetzung gemäß Anspruch 18, wobei in der ersten Beschichtung das Beschichtungsmittel ein Polymer oder Copolymer von Acrylat und/oder Methacrylat mit Ammoniumgruppen ist; der oberflächenaktive Stoff Natriumlaurylsulfat oder Polysorbat 80 ist; der Weichmacher Stearinsäure oder Dibutylsebacat oder Acetyltributylcitrat ist; und das Gleitmittel Magnesiumstearat oder Talkum ist; und wobei in der trennenden Beschichtung das Beschichtungsmittel Hydroxypropylcellulose ist; der Weichmacher Polyethylenglycol ist; und das Gleitmittel Talkum ist;
und wobei in der dritten Beschichtung der oberflächenaktive Stoff Natriumlaurylsulfat ist; der Weichmacher Stearinsäure ist; und das Gleitmittel Magnesiumstearat ist.

20. Pharmazeutische Zusammensetzung, welche eine fertige Darreichungsform ist, welche eine pharmazeutische Zusammensetzung gemäß irgendeinem der vorherigen Ansprüche umfasst, welche in Gelatine- oder HPMC-Kapseln gefüllt ist.

21. Verwendung einer Beschichtung, welche ein Polymer oder Copolymer von Acrylat und/oder Methacrylat mit Ammoniumgruppe, und/oder Chitosan oder Chitosanderivate umfasst, **gekennzeichnet durch** eine pH-abhängige Auflösung, welche bei sauren Bedingungen (pH < 5,5) schnell ist und sehr langsam bei höheren pH-Werten ist, um den Effekt des erhöhten pH-Werts auf die Freisetzung und/oder der Stabilität eines Medikaments und/oder von Medikamentformulierungen mit pH-abhängiger Auflösung und/oder Stabilität, hervorgerufen **durch** die gleichzeitige Verwendung von Antaziden, zu neutralisieren, wobei das Medikament Tolterodin oder ein Salz davon ist.

22. Verwendung von Beschichtung gemäß Anspruch 21 bei der Herstellung eines Medikaments, welches einen erhöhten Cmax umfasst, wenn es gleichzeitig mit einem Antazid verabreicht wird.

23. Verwendung von Beschichtung gemäß Anspruch 22 bei der Herstellung eines Medikaments zur Behandlung von Harnwegserkrankungen oder gastrointestinalen Erkrankungen oder Bluthochdruck.

24. Verwendung von Beschichtung gemäß vorherigem Anspruch bei der Herstellung eines Medikaments, welches Tolterodin-Tartrat umfasst.

25. Verwendung eines Copolymers von Acrylat und Methacrylat mit Ammoniumgruppe zur Beschichtung einer pharmazeutischen Zusammensetzung, welche Tolterodin umfasst.

26. Verfahren zur Herstellung einer pharmazeutischen Zusammensetzung, **dadurch gekennzeichnet, dass** es aus den folgenden Schritten besteht:
a) Herstellung von Kernen, welche einen aktiven pharmazeutischen Bestandteil umfassen, wobei der aktive pharmazeutisch Bestandteil Tolterodin oder ein Salz davon ist;
b) Auftragen einer Beschichtung auf die Kerne, wobei die Beschichtung ein Polymer oder Copolymer von Acrylat und/oder Methacrylat mit Ammoniumgruppe und/oder Chitosan oder Chitosanderivate umfasst, **dadurch gekennzeichnet, dass** die Beschichtungsschicht die rasche Auflösung bei einem pH < 5,5 und eine langsame Auflösung bei höheren pH-Werten bereitstellt.

27. Verfahren gemäß dem vorherigen Anspruch, wobei die aktive pharmazeutische Bestandteil Tolterodin oder Tolterodin-Tartrat ist und die Beschichtung, welche in Schritt b) aufgetragen wird, Aminoalkylmethacrylat-Copolymere und/oder Chitosan oder Chitosanderivate umfasst.

28. Verfahren zur Herstellung einer pharmazeutischen Zusammensetzung, **dadurch gekennzeichnet, dass** es aus den folgenden Schritten besteht:
a) Herstellung von Kernen, welche eine aktive pharmazeutische Bestandteil umfassen, wobei die aktive pharmazeutische Bestandteil Tolterodin oder ein Salz davon ist;
b) Auftragen einer magensaftresistenten Beschichtung oder eines Mittels, welches die Freisetzung steuert, auf die Kerne;
c) Auftragen einer zweiten trennenden Beschichtung auf die ersten beschichteten Kerne, welches zweite Kerne ergibt;
d) Auftragen einer dritten Beschichtung auf die zweiten Kerne, wobei die Beschichtung ein Polymer oder Copolymer von Acrylat und/oder Methacrylat mit Ammoniumgruppe und/oder Chitosan oder Chitosanderivate umfasst, **dadurch gekennzeichnet, dass** die dritte Beschichtungsschicht die schnelle Auflösung bei einem
pH < 5,5 und eine langsame Auflösung bei höheren pH-Werten bereitstellt.

29. Verfahren gemäß Anspruch 28, wobei die Beschichtung in Schritt d) ein Polymer umfasst, welches ein Aminoalkylmethacrylat-Copolymer ist.

30. Verfahren gemäß Anspruch 29, wobei das Aminoalkylmethacrylat-Copolymer ein Copolymer von Acrylat und Methacrylat mit Ammoniumgruppe ist.

31. Verfahren gemäß einem der Ansprüche 28 bis 30, wobei die Beschichtung in Schritt c) Hydroxypropylcellulose umfasst.

32. Verfahren gemäß einem der Ansprüche 28 bis 31, wobei die aktive pharmazeutische Bestandteil Tolterodin-Tartrat ist.

33. Zusammensetzung gemäß einem der Ansprüche 1 bis 20 zur Verwendung zu einer Behandlung eines Patienten, der gleichzeitig mit Antazid behandelt wird.

34. Pharmazeutische Zusammensetzung gemäß Anspruch 1 zur Verwendung als ein Medikament bei einer Behandlung eines Patienten, der gleichzeitig mit Antazid behandelt wird.

35. Pharmazeutische Zusammensetzung zur Verwendung gemäß Anspruch 34, wobei das Medikament zur Behandlung von Urinierproblemen ist.

36. Pharmazeutische Zusammensetzung zur Verwendung gemäß Anspruch 35, wobei Urinierschwierigkeiten häufiges Urinieren und/oder eine Unfähigkeit, den Urin zu kontrollieren, ist.

37. Zusammensetzung gemäß einem der Ansprüche 1 bis 20 zur Verwendung bei der Behandlung von Harnwegskrankheiten.

38. Zusammensetzung zur Verwendung gemäß Anspruch 37, wobei die Harnwegskrankheit ein Urinierproblem ist.

39. Zusammensetzung zur Verwendung gemäß Anspruch 38, wobei das Urinierproblem häufiges Urinieren und/oder die Unfähigkeit, das Urinieren zu steuern, ist.

40. Zusammensetzung gemäß einem der Ansprüche 1 bis 20 zur Verwendung in der Verabreichung eines krampflösenden Medikaments der Harnwege, wobei die Verabreichung gleichzeitig mit der Verabreichung eines Antazids durchgeführt wird.

## Revendications

1. Composition pharmaceutique comprenant un ingrédient pharmaceutique actif, dans laquelle l'ingrédient pharmaceutique actif est de la toltérodine ou un sel de celle-ci, ladite composition comportant un revêtement comprenant un polymère ou copolymère d'acrylate et/ou de méthacrylate avec un groupe ammonium, et/ou du chitosane ou des dérivés de chitosane, **caractérisée par** la dissolution rapide à des conditions acides (pH < 5,5) et la dissolution lente à des valeurs de pH supérieures.

2. Composition pharmaceutique selon la revendication 1, comprenant du tartrate de toltérodine, ladite composition comportant un revêtement présentant une dissolution en fonction du pH, **caractérisée par** la dissolution rapide à un pH < 5,5 et la dissolution lente à des valeurs de pH supérieures.

3. Composition pharmaceutique selon la revendication 2, dans laquelle le revêtement comprend un polymère ou copolymère d'acrylate et/ou de méthacrylate avec un groupe ammonium.

4. Composition pharmaceutique selon la revendication 3, dans laquelle le revêtement comprend un polymère ou copolymère de méthacrylate de diméthylamino-éthyle.

5. Composition pharmaceutique selon l'une quelconque des revendications 1 à 4, dans laquelle la dissolution rapide à un pH < 5,5 est définie par le fait que, à un pH de 2,0, le revêtement est complètement dissous en moins de 30 minutes s'il est soumis au test de dissolution dans un dispositif 2 selon la norme USP 28.

6. Composition pharmaceutique selon l'une quelconque des revendications 1 à 4, dans laquelle la dissolution lente à des valeurs de pH supérieures à 5,5 est définie par le fait que, à un pH de 6,8, le revêtement n'est sensiblement pas dissous après 180 minutes, s'il est soumis au test de dissolution dans un dispositif 2 selon la norme USP 28.

7. Composition pharmaceutique selon l'une quelconque des revendications 1 à 4, **caractérisée en ce que** moins de 20% en poids de l'ingrédient pharmaceutique actif est libéré à un pH = 6,8 en moins de 3h s'il est soumis au test de dissolution dans un dispositif 2 selon la norme USP 28.

8. Composition pharmaceutique selon l'une quelconque des revendications 1 à 4, **caractérisée en ce que**, dans la solution ou la suspension, moins de 20% en poids de la quantité de polymère utilisée dans le revêtement est détectée après 3h, lorsque la composition est soumise au test de dissolution dans un dispositif 2 selon la norme USP 28.

9. Composition pharmaceutique selon la revendication 8, dans laquelle la quantité de polymère est détectée par chromatographie HPLC en solution ou dispersion après avoir soumis la composition au test de dissolution dans un dispositif 2 selon la norme USP 28.

10. Composition pharmaceutique selon l'une quelconque des revendications précédentes, **caractérisée en ce qu'**elle consiste en noyaux revêtus par un premier revêtement, dans laquelle les noyaux comprennent ledit ingrédient pharmaceutique actif, et un deuxième revêtement de séparation ainsi qu'un troisième revêtement comprenant un copolymère d'acrylate et de méthacrylate avec un groupe ammonium.

11. Composition pharmaceutique selon la revendication précédente, dans laquelle le deuxième revêtement comprend de la cellulose hydroxypropyle.

12. Composition pharmaceutique selon les revendications 10 ou 11, dans laquelle les noyaux sont des pastilles.

13. Composition pharmaceutique selon la revendication 10 ou 11, dans laquelle les noyaux sont des noyaux inertes revêtus d'un film comprenant un ingrédient pharmaceutique actif.

14. Composition pharmaceutique, **caractérisée en ce qu'**elle consiste en pastilles qui comprennent du tartrate de toltérodine qui sont enrobées par un ou éventuellement plusieurs revêtements et au moins l'un desdits revêtements comprend un polymère ou copolymère d'acrylate et/ou de méthacrylate avec des groupes ammonium et/ou du chitosane ou des dérivés de chitosane, le revêtement étant **caractérisé par** une dissolution rapide à des conditions acides (pH < 5,5) et une dissolution lente à des valeurs de pH supérieures.

15. Composition pharmaceutique selon la revendication 14, dans laquelle ledit polymère ou copolymère est un copolymère Eudragit E PO ou un copolymère Eudragit E 100.

16. Composition pharmaceutique, **caractérisée en ce qu'**elle consiste en noyaux de pastille comprenant du tartrate de toltérodine enrobé avec un premier revêtement, un deuxième revêtement et un troisième revêtement comprenant de l'Eudragit E PO, du laurylsulfate de sodium, de l'acide stéarique, et du stéarate de magnésium, le revêtement étant **caractérisé par** une dissolution rapide à des conditions acides (pH < 5,5) et une dissolution lente à des valeurs de pH supérieures.

17. Composition pharmaceutique selon la revendication 16, **caractérisée en ce qu'**un deuxième revêtement comprend de l'éthylcellulose, et/ou de la cellulose hydroxypropyle.

18. Composition pharmaceutique comprenant des noyaux dans laquelle les noyaux comprennent du tartrate de toltérodine,
et un premier revêtement qui comprend :
de 6 à 12 % d'agent de revêtement ;
de 0,5 à 1,5 % de surfactant ;
de 0, 5 à 1,5 % d'agent plastifiant ; et
de 2 à 5 % d'agent glissant ;
et un revêtement de séparation qui comprend :
de 2 à 5 % d'agent de revêtement ;
de 0,2 à 0,5 % d'agent plastifiant ; et
de 0,5 à 1 % d'agent glissant ;
et un troisième revêtement comprenant :
de 6 à 12 % d'agent de revêtement, qui est un polymère ou copolymère d'acrylate et/ou de méthacrylate avec des groupes ammonium ;
de 0,5 à 1,5 % de surfactant ;
de 0,5 à 1,5 % d'agent plastifiant ; et
de 2 à 5% d'agent glissant ;
de telle sorte que les quantités représentent des valeurs en poids de la composition finale, et dans laquelle le troisième revêtement est **caractérisé par** une dissolution rapide à des conditions acides (pH < 5,5) et une dissolution lente à des valeurs de pH supérieures.

19. Composition pharmaceutique selon la revendication 18,
dans laquelle, dans le premier revêtement, l'agent de revêtement est un polymère ou copolymère d'acrylate et/ou de méthacrylate avec des groupes ammonium ; le surfactant est du laurylsulfate de sodium ou du polysorbate 80 ; l'agent plastifiant est de l'acide stéarique ou du sébaçate de dibutyle ou du citrate d'acétyltributyle ; et l'agent glissant est du stéarate de magnésium ou du talc ;
et dans laquelle, dans le revêtement de séparation, l'agent de revêtement est de la cellulose hydroxypropyle ; l'agent plastifiant est du glycol de polyéthylène ; et l'agent glissant est du talc ;
et dans laquelle, dans le troisième revêtement, le surfactant est du laurylsulfate de sodium ; l'agent plastifiant est de l'acide stéarique ; et l'agent glissant est du stéarate de magnésium.

20. Composition pharmaceutique qui est une forme posologique finie comprenant la composition pharmaceutique selon l'une quelconque des revendications précédentes remplissant des capsules de gélatine ou de HPMC.

21. Utilisation d'un revêtement comprenant un polymère ou copolymère d'acrylate et/ou de méthacrylate avec des groupes ammonium, et/ou du chitosane ou des dérivés de chitosane, **caractérisée par** une dissolution en fonction du pH qui est rapide à des conditions acides (pH < 5,5) et très lente à des valeurs de pH supérieures afin de neutraliser l'effet du pH élevé sur la libération et/ou la stabilité d'un médicament et/ou de formulations médicamenteuses qui présentent une dissolution et/ou une stabilité en fonction du pH, provoquée par l'utilisation simultanée d'antiacides, dans laquelle le médicament est de la toltérodine ou un sel de celle-ci.

22. Utilisation de revêtement selon la revendication 21, lors de la fabrication d'un médicament présentant une valeur Cmax accrue s'il est administré simultanément à des antiacides.

23. Utilisation de revêtement selon la revendication 22, lors de la fabrication d'un médicament pour le traitement de la maladie urinaire ou gastro-intestinale ou l'hypertension.

24. Utilisation de revêtement selon la revendication précédente pour la fabrication d'un médicament comprenant du tartrate de toltérodine.

25. Utilisation d'un copolymère d'acrylate et de méthacrylate avec des groupes ammonium afin d'enrober une composition pharmaceutique comprenant de la toltérodine.

26. Procédé de fabrication d'une composition pharmaceutique **caractérisé en ce qu'**il comprend les étapes suivantes :
a) préparation de noyaux comportant un ingrédient pharmaceutique actif, dans lequel l'ingrédient pharmaceutique actif est de la toltérodine ou un sel de celle-ci ;
b) application d'un revêtement sur lesdits noyaux, où ledit revêtement comprend un polymère ou copolymère d'acrylate et/ou de méthacrylate avec des groupes ammonium, et/ou du chitosane ou des dérivés chitosane, **caractérisé en ce que** la couche de revêtement assure la dissolution rapide à un pH < 5,5 et une dissolution lente à des valeurs de pH supérieures.

27. Procédé selon la revendication précédente, dans lequel l'ingrédient pharmaceutique actif est de la toltérodine ou du tartrate de toltérodine et le revêtement appliqué dans l'étape b) comprend des copolymères de méthacrylate d'aminoalkyle et/ou du chitosane ou des dérivés de chitosane.

28. Procédé de fabrication d'une composition pharmaceutique **caractérisé en ce qu'**il comprend les étapes suivantes :
a) préparation de noyaux comprenant un ingrédient pharmaceutique actif, où l'ingrédient pharmaceutique actif est de la toltérodine ou un sel de celle-ci ;
b) application d'un revêtement entérique ou agent de contrôle de libération sur lesdits noyaux ;
c) application d'un deuxième revêtement de séparation sur lesdits premiers noyaux revêtus, conduisant à des deuxièmes noyaux ;
d) application d'un troisième revêtement sur lesdits deuxièmes noyaux, dans lequel ledit revêtement comprend un polymère ou copolymère d'acrylate et/ou de méthacrylate avec des groupes ammonium, et/ou du chitosane ou des dérivés de chitosane, **caractérisé en ce que** la troisième couche de revêtement assure la dissolution rapide à un pH < 5,5 et une dissolution lente à des valeurs de pH supérieures.

29. Procédé selon la revendication 28 dans lequel le revêtement à l'étape d) comprend un polymère, qui est un copolymère de méthacrylate d'aminoalkyle.

30. Procédé selon la revendication 29, dans lequel le copolymère de méthacrylate d'aminoalkyle est un copolymère d'acrylate et de méthacrylate avec un groupe ammonium.

31. Procédé selon l'une quelconque des revendications 28 à 30, dans lequel le revêtement à l'étape c) comprend de la cellulose hydroxypropyle.

32. Procédé selon l'une quelconque des revendications 28 à 31, dans lequel l'ingrédient pharmaceutique actif est du tartrate de toltérodine.

33. Composition selon l'une quelconque des revendications 1 à 20, destinée à une utilisation dans le traitement d'un patient qui est traité simultanément avec un antiacide.

34. Composition pharmaceutique selon la revendication 1, destinée à une utilisation comme médicament dans un traitement de patient qui est traité simultanément avec un antiacide.

35. Composition pharmaceutique destinée à une utilisation selon la revendication 34, dans laquelle le médicament est destiné au traitement d'une affection urinaire.

36. Composition pharmaceutique destinée à une utilisation selon la revendication 35, dans laquelle l'affection urinaire est le besoin fréquent d'uriner et/ou l'incontinence.

37. Composition selon l'une quelconque des revendications 1 à 20, destinée à une utilisation pour le traitement des maladies urinaires.

38. Composition destinée à une utilisation selon la revendication 37, dans laquelle la maladie urinaire est une affection urinaire.

39. Composition destinée à une utilisation selon la revendication 38, dans laquelle l'affection urinaire est le besoin fréquent d'uriner et/ou l'incontinence.

40. Composition selon l'une quelconque des revendications 1 à 20, destinée à une utilisation lors de l'administration d'un médicament antispasmodique urinaire, dans laquelle l'administration est assurée de manière simultanée à l'administration d'un antiacide.
